# EUROPÄISCHE PATENTSCHRIFT

(11) **EP 2 230 182 B1**
(45) Veröffentlichungstag und Bekanntmachung des Hinweises auf die Patenterteilung: **09.05.2012**
(21) Anmeldenummer: 10002978.4
(22) Anmeldetag: 10.05.2000
(51) Int. Cl.: B65B 61/20, A61M 5/28, B23P 19/08, B65B 7/28, B65B 3/00

(54) **Vorrichtung zum Setzen eines Stopfens in einem Spritzenzylinder**
Device for setting a stopper in a syringe cylinder
Dispositif de mise en place d'un bouchon dans un cylindre de seringue

(30) Priorität: 29.07.1999 DE 19935681
(43) Veröffentlichungstag der Anmeldung: 22.09.2010
(62) Teilanmeldung aus: 00109845.8
(73) Patentinhaber: Arzneimittel GmbH Apotheker Vetter & Co. Ravensburg, 88212 Ravensburg (DE)
(72) Erfinder: Vetter, Udo J., 88214 Ravensburg (DE); Steinbach, Klaus, 88255 Baienfurt (DE); Hecht, Anton, 88255 Baienfurt (DE)
(74) Vertreter: Dziewior, Joachim

(56) Entgegenhaltungen:
- US-A- 3 470 671
- US-A- 3 730 235

## Beschreibung

Die Erfindung betrifft eine Vorrichtung zum Setzen eines Stopfens in einem Spritzenzylinder, mit einer Zylinderhülse, die an einem Aufnahmekopf angeordnet ist und in axialer Richtung ins Lumen des Spritzenzylinders einfahrbar ist und die in ihrem Inneren den zu setzenden Stopfen trägt, ferner mit einer zur Zylinderhülse koaxial angeordneten und dieser gegenüber axial verschiebbaren Zentrierhülse, die an ihrem dem Spritzenzylinder zugewandten Ende eine konische Zentrierfläche für die Stirnseite des Spritzenzylinders aufweist, wobei das freie Ende der Zentrierhülse in der Ausgangsstellung zumindest geringfügig über das Ende der Zylinderhülse vorsteht.

Eine derartige Vorrichtung ist in unterschiedlichen Ausführungsformen aus dem Stand der Technik (z.B. US 3470671 A und US 3730235 A) bekannt und kommt unter anderem beim Abfüllvorgang von Fertigspritzen, insbesondere von Zweikammerspritzen, zum Einsatz. Der Vorteil von Zweikammerspritzen besteht insbesondere darin, pharmazeutische Substanzen, die in wässriger Lösung instabil sind, gleichwohl in vorgefüllter Form zur Applikation anbieten zu können, indem die eigentliche pharmazeutische Substanz in Pulverform getrennt von dem Lösungsmittel - jeweils in eigenen Kammern - abgefüllt wird. Um der pharmazeutischen Substanz das Lösungsmittel zu entziehen, wird üblicherweise ein Gefriertrocknungsprozeß angewandt. Die beiden Kammern der Spritze sind gewöhnlich durch einen Mittelstopfen voneinander abgetrennt.

Vor der Anwendung der Spritze wird die Lösung aus der einen Kammer über den sogenannten by-pass in die kanülenseitige Kammer überführt, so daß eine Vermischung beider Substanzen bzw. eine Rekonstitution der pulverförmigen Substanz erfolgen kann.

Die Zweikammerspritze besteht in der Regel aus einem Glaszylinder, dessen kanülenseitige Öffnung halsförmig verengt ausgebildet und entsprechend dem aufzunehmenden Verschlußsystem geformt ist. Das proximale Ende dient der Aufnahme einer Fingerauflage und ist entsprechend mit einem Rollrand ausgebildet.

Vor dem Befüllen der Spritze mit den erwähnten Produkten müssen die beiden Kammern durch Setzen des Stopfens kurz vor dem by-pass abgetrennt werden. Das Setzen eines Stopfens erfolgt üblicherweise durch ein dünnwandiges Setzrohr, dessen Außendurchmesser geringfügig kleiner ist als der lichte Innendurchmesser des Glaszylinders. Sobald der Stopfen an die axial vorgesehene Position gebracht ist, wird der Stopfen möglicherweise durch einen Stempel herausgedrückt, worauf er nach entsprechender Expansion an der Glaszylinder-Innenwand zur Anlage kommt.

Anschließend erfolgt das Befüllen der vorderen Kammer mit dem flüssigen Produkt. Hierbei fährt eine Füllnadel einer Abfüllvorrichtung durch den Hals des Glaszylinders bis kurz vor den Mittelstopfen hinein. Anschließend erfolgt während gleichzeitigem Zurückziehen der Füllnadel die Befüllung der vorderen Kammer.

Sowohl beim Einfahren des Setzrohres von dem proximalen Ende her wie auch beim Einführen der Füllnadel durch die Halsmündung des Glaszylinders ist eine Zentrierung der als Füllnadel bzw. Setzrohr dienenden Zylinderhülse erforderlich, um beim Einfahren in den Spritzenzylinder Beschädigungen an dessen stirnseitigem Ende zu vermeiden.

Der Erfindung liegt die Aufgabe zugrunde, eine Vorrichtung der eingangs genannten Art zu schaffen, durch die das Setzen des Stopfens, das grundsätzlich auch mittels eines Stempels durch das Zylinderrohr hindurch möglich ist, bevorzugt durch Vakuum erfolgen kann, mit dem der Spritzenzylinder kurzzeitig beaufschlagt wird, wodurch der Stopfen aus der Zylinderhülse "herausgezogen" wird. Zusätzlich soll dabei sichergestellt sein, daß beim Einfahren der Zylinderhülse in die Spritze eine ausreichende koaxiale Ausrichtung zwischen dem Spritzenzylinder und der Zylinderhülse gewährleistet ist, so daß Beschädigungen des Spritzenzylinders und insbesondere auch der Zylinderhülse sicher ausgeschlossen sind.

Eine diese Aufgabe lösende Vorrichtung ist dadurch gekennzeichnet, daß zwischen der Zentrierhülse und der Zylinderhülse ein Ringspalt angeordnet ist, der mit einem Absauganschluß in Verbindung steht.

Der durch die Erfindung erreichte Vorteil besteht im wesentlichen darin, daß, nachdem zunächst an dem Spritzenzylinder mit der Zentrierfläche angreifenden Zentrierhülse die Zylinderhülse und der Spritzenzylinder in eine zueinander fluchtende Position gebracht wurden, so daß im Anschluß daran die Zylinderhülse in den Spritzenzylinder einfahren kann, ohne daß - zumindest stirnseitig - beide miteinander in Kontakt kommen, also ein gegenseitiges Aufsitzen mit der Folge von Schäden vermieden wird, anschließend auf einfache Weise der Stopfen gesetzt werden kann.

Nach einer ersten vorteilhaften Ausgestaltung der Erfindung, die sich insbesondere bei Spritzenzylindern mit einer radial nach außen vorstehenden Rollrand oder einer Fingerauflage eignet, ist die Zentrierfläche als Außenkonusfläche ausgebildet.

Ist dagegen keine Fingerauflage vorhanden, so ist es besonders vorteilhaft, wenn die Zentrierfläche als Innenkonusfläche ausgebildet ist, da dann der Konus nicht ins Innere des Spritzenzylinders greift, dessen lichter Innenquerschnitt also keine Querschnittsreduzierung erfährt.

Da bei der als Außenkonus ausgestalteten Zentrierfläche ohne allzu große Querschnittsreduzierung des lichten Innenquerschnitts des Spritzenzylinders nur in geringem Maße eine Zentrierung möglich ist, kann es von Vorteil sein, wenn die Zentrierhülse zusätzlich einen Zentrierkragen mit einer Innenkonusfläche aufweist. Dieser Zentrierkragen bewirkt dann zunächst eine Vorzentrierung, bis die eigentliche Zentrierfläche zur Wirkung kommt.

Weiter ist es im Rahmen der Erfindung vorgesehen, daß die Zylinderhülse am Aufnahmekopf feststehend angeordnet ist und die Zentrierhülse gegen die Kraft einer Feder zum Aufnahmekopf hin verstellbar ist. Den eigentlichen Bewegungshub macht also der Aufnahmekopf, wobei zunächst die Zentrierhülse am Spritzenzylinder zur Anlage kommt und dann - nach der sich anschließenden Zentrierung - beim weiteren Bewegungshub des Aufnahmekopfes die Zylinderhülse in den Spritzenzylinder einfährt. Dann kann die als Setzrohr gestaltete Zylinderhülse den Stopfen setzen.

Für diesen Bewegungsablauf ist die Zentrierhülse vorteilhafterweise in einer Außenhülse des Aufnahmekopfs verschiebbar geführt.

Ergänzend kann die Zentrierhülse an ihrer Innen- und/oder Außenmantelfläche mit mindestens einer Längsnut als Strömungskanal versehen sein, wodurch die Effektivität des Absaugens noch erhöht werden kann.

Schließlich ist es für diese Anwendungen günstig, wenn die Zentrierfläche als Dichtfläche ausgebildet ist.

Im folgenden wird die Erfindung an in der Zeichnung dargestellten Ausführungsbeispielen näher erläutert; es zeigen:
- Fig. 1: den Gegenstand der Erfindung zum Setzen von Stopfen an rein zylindrischen Spritzenkörpern,
- Fig. 2: eine nur teilweise Darstellung des Gegenstandes von Fig. 1 in modifizierter Form zum Setzen von Stopfen an Spritzenzylindern mit Rollrand,
- Fig. 3: eine alternative Ausgestaltung des Gegenstandes nach Fig. 2,
- Fig. 4: den Gegenstand nach Fig. 2, nach erfolgter Zentrierung,
- Fig. 5: den Gegenstand nach Fig. 1, nach erfolgter Zentrierung,
- Fig. 6: den Gegenstand nach Fig. 4, jedoch mit eingefahrenem Setzrohr,
- Fig. 7: den Gegenstand nach Fig. 5, jedoch mit eingefahrenem Setzrohr.

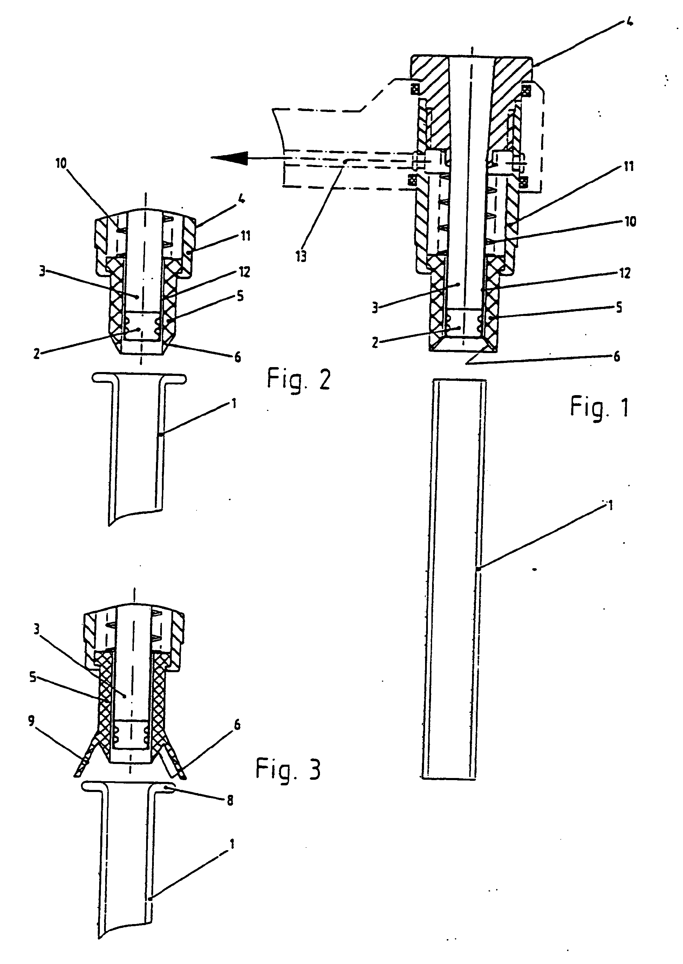

Die in der Zeichnung in den Fig. 1 bis 7 dargestellte Vorrichtung dient zum Setzen eines Stopfens 2 im Spritzenzylinder 1.

Im einzelnen weist die Vorrichtung eine Zylinderhülse 3 auf, die an einem Aufnahmekopf 4 angeordnet ist und in axialer Richtung ins Lumen des Spritzenzylinders 1 einfahrbar ist.

Die Zylinderhülse 3 ist als Setzrohr ausgebildet, das in seinem Inneren endseitig den zu setzenden Stopfen 2 trägt.

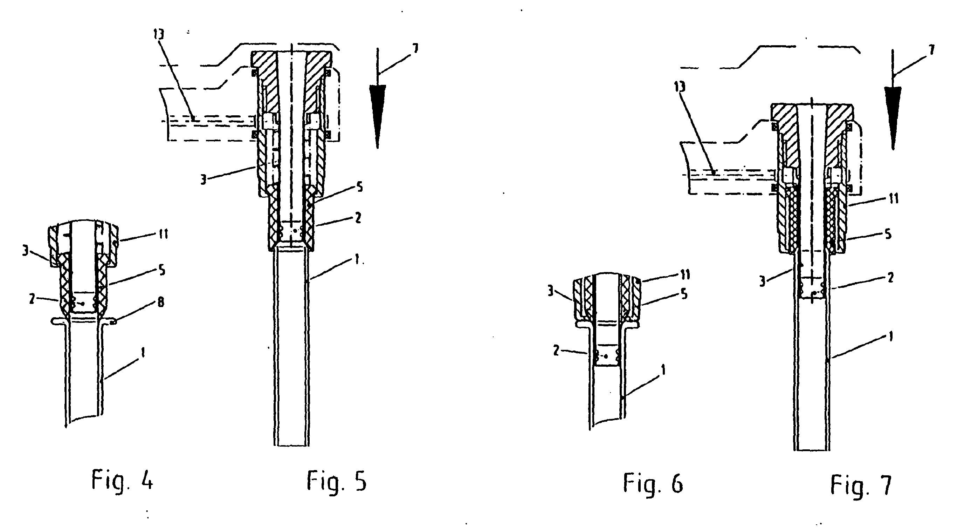

Koaxial zur Zylinderhülse 3 ist eine Zentrierhülse 5 angeordnet, die gegenüber der Zylinderhülse 3 axial verschiebar ist. Diese Zentrierhülse 5 weist an ihrem dem Spritzenzylinder 1 zugewandten Ende eine konische Zentrierfläche 6 für die Stirnseite des Spritzenzylinders 1 auf. In der Ausgangslage, wie sie in den Fig. 1 bis 3 dargestellt ist, steht das freie Ende der Zentrierhülse 5 zumindest geringfügig über das Ende der Zylinderhülse 3 vor, so daß beim Heranfahren des Aufnahmekopfes 4 an den Spritzenzylinder 1, wie dies in den Fig. 5 und 7 durch den Pfeil 7 dargestellt ist, zunächst die Konusfläche der Zentrierhülse 5 mit dem Spritzenzylinder 1 in Kontakt kommt.

In der Fig. 1 ist die Zentrierfläche 6 als Innenkonusfläche ausgebildet, die insbesondere für rein zylindrische Spritzen vorteilhaft ist, da hier die Konusfläche verhältnismäßig groß gestaltet werden kann und somit ein großer Zentrierbereich erreicht wird.

In der Fig. 2 ist die Zentrierfläche 6 dagegen als Außenkonusfläche ausgebildet, so daß hier der Konus geringfügig ins Innere des Spritzenzylinders 1 vorsteht und den lichten Öffnungsquerschnitt des Spritzenzylinders 1 entsprechend verringert. Diese Außenkonusfläche bietet sich dann an, wenn der Spritzenzylinder 1, wie dargestellt, mit einem Rollrand 8 bzw. einer Fingerauflage versehen ist, da dessen Außenkontur in der Regel größeren Toleranzen unterworfen ist. Naturgemäß ist dadurch der Zentrierbereich erheblich eingeschränkt.

Hierfür bietet sich jedoch die Ausführungsform nach Fig. 3 an, bei der die Zentrierhülse 5 zusätzlich einen Zentrierkragen 9 mit einer Innenkonusfläche aufweist, der eine Vorjustierung des Spritzenzylinders 1 über die Außenkontur des Rollrandes 8 vornimmt.

Die Zylinderhülse 3 ist am Aufnahmekopf 4 feststehend angeordnet, während die Zentrierhülse 5 gegen die Kraft einer Feder 10 zum Aufnahmekopf 4 hin verstellbar ist. Auf diese Weise ist einerseits sichergestellt, daß die Zentrierhülse 5 unter Federkraft der Stirnfläche des Spritzenzylinders 1 anliegt und somit für eine fortwährende Zentrierung sorgt, andererseits gilt die Zentrierhülse 5 nach erfolgtem Setzen des Stopfens 2 bzw. Befüllens der Spritze selbsttätig in die Ausgangsposition zurück, so daß sich sofort der nächste Arbeitsschritt anschießen kann. Die Zentrierhülse 5 ist dazu in einer Außenhülse 11 des Aufnahmekopfes 4 verschiebar geführt, wie sich dies unmittelbar aus den Zeichnungen ergibt.

Zwischen der Zentrierhülse 5 und der Zylinderhülse 3 ist ein Ringspalt 12 vorgesehen, der mit einem Absauganschluß 13 in Verbindung steht. Dadurch besteht die Möglichkeit, zunächst Partikel durch Absaugen zu entfernen, die z.B. aus dem Bewegungshub der Zentrierhülse 5 entstehen können. Beim Setzen von Stopfen 2 besteht hierdurch die Möglichkeit, im Spritzenzylinder 1 einen Unterdruck zu erzeugen, wodurch der Stopfen 2 aus dem Setzrohr 3 herausgezogen wird.

Um hier eine Verbesserung der Saugleistung zu erreichen, kann die Zentrierhülse 5 an ihrer Innen- oder Außenmantelfläche mit mindestens einer Längsnut 14 als Strömungskanal versehen sein.

Um hierbei Druckverluste zu vermeiden, kann die Zentrierfläche 6 in üblicher Weise, also etwa durch geringfügig elastische Gestaltung, als Dichtfläche ausgebildet sein.

## Patentansprüche

1. Vorrichtung zum Setzen eines Stopfens (2) in einem Spritzenzylinder (1), mit einer Zylinderhülse (3), die an einem Aufnahmekopf (4) angeordnet ist und in axialer Richtung ins Lumen des Spritzenzylinders (1) einfahrbar ist und die in ihrem Inneren den zu setzenden Stopfen (2) trägt, ferner mit einer zur Zylinderhülse (3) koaxial angeordneten und dieser gegenüber axial verschiebbaren Zentrierhülse (5), die an ihrem dem Spritzenzylinder (1) zugewandten Ende eine konische Zentrierfläche (6) für die Stirnseite des Spritzenzylinders (1) aufweist, wobei das freie Ende der Zentrierhülse (5) in der Ausgangsstellung zumindest geringfügig über das Ende der Zylinderhülse (3) vorsteht, **dadurch gekennzeichnet, daß** zwischen der Zentrierhülse (5) und der Zylinderhülse (3) ein Ringspalt (12) vorgesehen ist, der mit einem Absauganschluß (13) in Verbindung steht.

2. Vorrichtung nach Anspruch 1, **dadurch gekennzeichnet, daß** die Zentrierfläche (6) als Außenkonusfläche ausgebildet ist.

3. Vorrichtung nach Anspruch 1, **dadurch gekennzeichnet, daß** die Zentrierfläche (6) als Innenkonusfläche ausgebildet ist.

4. Vorrichtung nach Anspruch 2, **dadurch gekennzeichnet, daß** die Zentrierhülse (5) zusätzlich einen Zentrierkragen (9) mit einer Innenkonusfläche aufweist.

5. Vorrichtung nach einem der Ansprüche 1 bis 4, **dadurch gekennzeichnet, daß** die Zylinderhülse (3) am Aufnahmekopf (4) feststehend angeordnet ist und die Zentrierhülse (5) gegen die Kraft einer Feder (10) zum Aufnahmekopf (4) hin verstellbar ist.

6. Vorrichtung nach Anspruch 5, **dadurch gekennzeichnet, daß** die Zentrierhülse (5) in einer Außenhülse (11) des Aufnahmekopfs (4) verschiebbar geführt ist.

7. Vorrichtung nach einem der Ansprüche 1 bis 6, **dadurch gekennzeichnet, daß** die Zentrierhülse (5) an ihrer Innen- und/oder Außenmantelfläche mit mindestens einer Längsnut (14) als Strömungskanal versehen ist.

8. Vorrichtung nach einem der Ansprüche 1 bis 7, **dadurch gekennzeichnet, daß** die Zentrierfläche (6) als Dichtfläche ausgebildet ist.

## Claims

1. Apparatus for placing a plug (2) in a syringe cylinder (1) comprising a cylinder sleeve (3) which is arranged on a mounting head (4) and which can be introduced in the axial direction into the lumen of the syringe cylinder (1) and which in its interior carries the plug (2) to be placed, further comprising a centering sleeve (5) which is arranged coaxially relative to the cylinder sleeve (3) and which is axially displaceable with respect thereto and which at its end towards the syringe cylinder (1) has a conical centering surface (6) for the end of the syringe cylinder (1), wherein the free end of the centering sleeve (5) in the starting position projects at least slightly beyond the end of the cylinder sleeve (3), **characterised in that** provided between the centering sleeve (5) and the cylinder sleeve (3) is an annular gap (12) which is connected to a suction connection (13).

2. Apparatus according to claim 1 **characterised in that** the centering surface (6) is in the form of an external conical surface.

3. Apparatus according to claim 1 **characterised in that** the centering surface (6) is in the form of an internal conical surface.

4. Apparatus according to claim 2 **characterised in that** the centering sleeve (5) additionally has a centering collar (9) with an internal conical surface.

5. Apparatus according to one of claims 1 to 4 **characterised in that** the cylinder sleeve (3) is arranged stationarily on the mounting head (4) and the centering sleeve (5) is displaceable towards the mounting head (4) against the force of a spring (10).

6. Apparatus according to claim 5 **characterised in that** the centering sleeve (5) is guided displaceably in an external sleeve (11) of the mounting head (4).

7. Apparatus according to one of claims 1 to 6 **characterised in that** at its internal and/or external peripheral surface the centering sleeve (5) is provided with at least one longitudinal groove (14) as a flow passage.

8. Apparatus according to one of claims 1 to 7 **characterised in that** the centering surface (6) is in the form of a sealing surface.

## Revendications

1. Dispositif de mise en place d'un bouchon (2) dans un cylindre de seringue (1), avec une douille cylindrique (3) qui est disposée sur une tête de logement (4) et peut être introduite en direction axiale dans la tubulure du cylindre de seringue (1) et porte à l'intérieur le bouchon (2) à mettre en place, en outre avec une douille de centrage (5) disposée coaxialement à la douille cylindrique (3) et coulissant axialement par rapport à celle-ci, qui présente à son extrémité faisant face au cylindre de seringue (1) une surface de centrage conique (6) pour la face frontale du cylindre de seringue (1), l'extrémité libre de la douille de centrage (5) dépassant dans la position de départ au moins légèrement de l'extrémité de la douille cylindrique (3), **caractérisé en ce qu'**un interstice annulaire (12) est prévu entre la douille de centrage (5) et la douille cylindrique (3), lequel est en liaison avec un raccordement d'aspiration (13).

2. Dispositif suivant la revendication 1, **caractérisé en ce que** la surface de centrage (6) est conçue comme surface extérieure de cône.

3. Dispositif suivant la revendication 1, **caractérisé en ce que** la surface de centrage (6) est conçue comme surface intérieure de cône.

4. Dispositif suivant la revendication 2, **caractérisé en ce que** la douille de centrage (5) présente en supplément un rebord de centrage (9) avec une surface intérieure de cône.

5. Dispositif suivant l'une des revendications 1 à 4, **caractérisé en ce que** la douille cylindrique (3) est disposée à la tête de logement (4) de manière fixe et la douille de centrage (5) est mobile en direction de la tête de logement (4) contre la force d'un ressort (10).

6. Dispositif suivant la revendication 5, **caractérisé en ce que** la douille de centrage (5) est guidée de manière permettant le coulissement dans une douille extérieure (11) de la tête de logement (4).

7. Dispositif suivant l'une des revendications 1 à 6, **caractérisé en ce que** la douille de centrage (5) est pourvue à sa surface d'enveloppe intérieure et/ou extérieure d'au moins une rainure longitudinale (14) comme canal d'écoulement.

8. Dispositif suivant l'une des revendications 1 à 7, **caractérisé en ce que** la surface de centrage (6) est conçue comme surface d'étanchéité.
